# EUROPEAN PATENT APPLICATION

(11) **EP 3 623 034 A1**
(43) Date of publication of application: **18.03.2020**
(21) Application number: 18799121.1
(22) Date of filing: 27.04.2018
(51) Int. Cl.: B01D 53/14, C10L 3/10

(54) **DEVICE FOR REMOVING SULFUR-CONTAINING COMPOUND AND METHOD FOR REMOVING SULFUR-CONTAINING COMPOUND**

(30) Priority: 12.05.2017 JP 2017095352
(71) Applicant: Kuraray Co., Ltd., Kurashiki-shi, Okayama 710-0801 (JP)
(72) Inventor: TSURUTA, Takuo, Tokyo 100-8115 (JP); SHIMIZU, Masaki, Houston, Texas 77058 (US); SAITOU, Yuusuke, Kamisu-shi Ibaraki 314-0197 (JP)
(74) Representative: D Young & Co LLP
(86) International application number: PCT/JP2018/017143
(87) International publication number: WO 2018/207657

(57) **Abstract**

An apparatus for removing a sulfur-containing compound, including: a sulfur-containing compound removing part of bringing a gas to be treated containing a sulfur-containing compound into contact with an oil-soluble sulfur-containing compound absorbing agent, so as to remove the sulfur-containing compound; and a mixing part of mixing a sulfur-containing compound absorbed agent resulted from having the sulfur-containing compound absorbed to the sulfer-containing compound absorbing agent, and a non-aqueous liquid.

## Description

### Field of the Invention

The present invention relates to an apparatus for removing a sulfur-containing compound and a method for removing a sulfur-containing compound.

### Background of the Invention

Known methods for removing a sulfur-containing compound, which has been recognized as a problem due to the toxicity and offensive odor thereof and the potential of corrosion of devices thereby, from a fluid include a method using a metal oxide, such as zinc oxide (PTL 1), a method using a low molecular weight aldehyde, such as formalin and glyoxal, which is supplied as an aqueous solution (PTL 2), a method using a reversible absorbent, such as triethanolamine (PTL 3), and the like.

Among the aforementioned methods, the method using a metal oxide is recognized as a problem due to the generation of waste materials derived from the metal oxide, and the method using a low molecular weight aldehyde has a problem in compatibility with the fluid to be treated, a problem in waste water treatment, and the like. The method using a reversible absorbent has problems in utilities, excessive cost for plant construction, and the like since the absorbent is necessarily heated for regeneration thereof, and an additional treatment equipment is necessarily provided for treating hydrogen sulfide detached from the absorbent.

As a method for efficiently removing hydrogen sulfide, methods using an oil-soluble hydrogen sulfide remover have also been proposed (PTLs 4 and 5).

### Citation List

### Patent Literature

PTL 1: JP-A-1-307428
PTL 2: U.S. Patent No. 4,748,011
PTL 3: JP-A-1-304026
PTL 4: JP-T-2013-531728
PTL 5: WO 2016/121747

### Summary of the Invention

### Technical Problem

Although a sulfur-containing compound contained in a fluid can be removed by the aforementioned methods, there is still room for improvement in reduction of utilities required for treatment and waste materials. In mining sites of natural gas and crude oil, in particular, there are restrictions in measures for transporting waste materials to disposal sites and supply of utilities, and therefore a solution to the aforementioned problems is further demanded in the removal of a sulfur-containing compound from gas in these sites.

An object of the present invention is to provide an apparatus for removing a sulfur-containing compound capable of reducing the utilities required for the treatment and reducing the waste materials, in the removal of a sulfur-containing compound contained in a gas, and a method for removing a sulfur-containing compound using the apparatus.

### Solution to Problem

As a result of earnest investigations made by the present inventors, it has been found that the object can be achieved in such a manner that an apparatus for removing a sulfur-containing compound is provided with a mixing part of mixing a sulfur-containing compound absorbed agent resulted from having a sulfur-containing compound present in a gas to be treated absorbed to a sulfur-containing compound absorbing agent, and a non-aqueous liquid.

The present invention relates to the following items [1] to [5].
[1] An apparatus for removing a sulfur-containing compound, including:
   a sulfur-containing compound removing part of bringing a gas to be treated containing a sulfur-containing compound into contact with an oil-soluble sulfur-containing compound absorbing agent, so as to remove the sulfur-containing compound; and
   a mixing part of mixing a sulfur-containing compound absorbed agent resulted from having the sulfur-containing compound absorbed to the sulfur-containing compound absorbing agent, and a non-aqueous liquid.
[2] The apparatus for removing a sulfur-containing compound according to the item [1], wherein the non-aqueous liquid contains a hydrocarbon.
[3] The apparatus for removing a sulfur-containing compound according to the item [1] or [2], wherein the non-aqueous liquid is a liquid that is generated on mining of crude oil or natural gas.
[4] The apparatus for removing a sulfur-containing compound according to any one of the items [1] to [3], wherein the gas to be treated is a hydrocarbon that is in a gaseous state at ordinary temperature.
[5] The apparatus for removing a sulfur-containing compound according to any one of the items [1] to [4], additionally including a supplying device of supplying the gas to be treated being discharged from a mining site of crude oil or natural gas, or a refinery of crude oil or natural gas to the sulfur-containing compound removing part, and wherein the mixing part is included in a sulfur component recovery apparatus provided in the mining site or the refinery.
[6] A method for removing a sulfur-containing compound, including: removing a sulfur-containing compound from a gas to be treated containing the sulfur-containing compound, with the apparatus for removing a sulfur-containing compound according to any one of the items [1] to [5].

### Advantageous Effects of Invention

According to the apparatus for removing a sulfur-containing compound and the method for removing a sulfur-containing compound of the present invention, the utilities required for the treatment and the waste materials can be reduced, in the removal of a sulfur-containing compound contained in a gas.

### Detailed Description of the Invention

The apparatus for removing a sulfur-containing compound according to the present invention includes: a sulfur-containing compound removing part of bringing a gas to be treated containing a sulfur-containing compound into contact with an oil-soluble sulfur-containing compound absorbing agent, so as to remove the sulfur-containing compound; and a mixing part of mixing a sulfur-containing compound absorbed agent resulted from having the sulfur-containing compound absorbed to the sulfur-containing compound absorbing agent, and a non-aqueous liquid.

In the present invention, the "sulfur-containing compound" is at least one compound selected from the group consisting of hydrogen sulfide and compounds containing a -SH group. Specifically, the sulfur-containing compound may be only hydrogen sulfide, only a compound containing a -SH group, or a mixture thereof. The compound containing a -SH group is not particularly limited, and examples thereof include compounds that are classified to a mercaptan compound represented by the chemical formula, R-SH. Examples of the mercaptan compound include a compound, in which R is an alkyl group, such as methylmercaptan, ethylmercaptan, n-propylmercaptan, isopropylmercaptan, n-butylmercaptan, isobutylmercaptan, sec-butylmercaptan, tert-butylmercaptan, and n-amylmercaptan; a compound, in which R is an aryl group, such as phenylmercaptan; and a compound, in which R is an aralkyl group, such as benzylmercaptan.

In the present invention, the "total amount of active ingredients of the sulfur-containing compound absorbing agent" means the total amount of the components excluding the solvent when the sulfur-containing compound absorbing agent contains a solvent. When the sulfur-containing compound absorbing agent does not contain a solvent, the "total amount of active ingredients of the sulfur-containing compound absorbing agent" means the total amount of the sulfur-containing compound absorbing agent.

In the present invention, the "removal" of a sulfur-containing compound also encompasses reduction of the initial amount of the sulfur-containing compound in the gas to be treated, through conversion of the sulfur-containing compound to another compound, or other measures.

The converted compound after the conversion from the sulfur-containing compound to another compound by the sulfur-containing compound absorbing agent is discharged as a sulfur-containing compound absorbed agent from the sulfur-containing compound removing part, and as a result, the initial amount of the sulfur-containing compound in the gas to be treated is reduced.

The gas to be treated is not particularly limited, as far as the gas is in a gaseous state in the sulfur-containing compound removing part, and is typically a hydrocarbon that is in a gaseous state at ordinary temperature (for example, at ordinary temperature and ordinary pressure). Specific examples of the gas to be treated include natural gas, petroleum gas, oilfield gas, associated gas, tail gas, dimethyl ether, synthetic gas, and biogas. The gas to be treated may be only one kind or may be two or more kinds.

The sulfur-containing compound absorbing agent is not particularly limited, as far as the absorbing agent is oil-soluble. Examples of the sulfur-containing compound absorbing agent include a monoaldehyde, such as propionaldehyde, butanal, pentanal, hexanal, heptanal, octanal, nonanal, decanal, and benzaldehyde; an α,β-unsaturated aldehyde, such as acrolein, crotonaldehyde, and senecioaldehyde; a dialdehyde, such as 3-methylglutaraldehyde, 1,6-hexanedial, ethylpentanedial, 1,7-heptanedial, methylhexanedial, 1,8-octanedial, methylheptanedial, dimethylhexanedial, ethylhexanedial, 1,9-nonanedial, 2-methyl-1,8-octanedial, ethylheptanedial, 1,10-decanedial, dimethyloctanedial, ethyloctanedial, dodecanedial, hexadecanedial, 1,2-cyclohexanedicarbaldehyde, 1,3-cyclohexanedicarbaldehyde, 1,4-cyclohexanedicarbaldehyde, 1,2-cyclooctanedicarbaldehyde, 1,3-cyclooctanedicarbaldehyde, 1,4-cyclooctanedicarbaldehyde, and 1,5-cyclooctanedicarbaldehyde; a triazine compound having a hydrophobic alkyl side chain, such as methyl and tert-butyl, described in U.S. Patent No. 5,674,377, US-A-2011/130298, Canadian Patent No. 2,845,574, EP-A-0620266, and the like; a triazine compound having an alkoxyalkyl side chain, such as 3-methoxypropyl, 2-methoxyisopropyl, and 2-methyoxyethyl, described in U.S. Patent No. 5,347,004 and the like; a quaternary ammonium salt described in EP-A-0538819 and the like; a reaction product of a hindered amine and an aldehyde described in UK-A-2409859 and the like; a metal carboxylate salt described in U.S. Patent No. 6,599,472, US-A-2014/305845, EP-A-2792732, US-A-2017/022109, WO 2015/009429, and the like; a triazine compound produced from N,N,N',N'-tetramethylpropanediamine described in US-A-2003/089641, US-A-2007/284288, and the like; an α-aminoether described in US-A-2012/012507, WO 2012/009396, and the like; a reaction product of an aromatic aldehyde and an amine or an amine derivative described in US-A-2009/065445, Russian Patent No. 2,594,565, and the like; a triazine compound formed of an alkanolamine having from 1 to 6 carbon atoms and an aldehyde having from 1 to 6 carbon atoms described in U.S. Patent No. 8,512,449 and the like; a mixture of a metal salt, an oil-soluble amine, and an aldehyde compound described in WO 2013/181056 and the like; a hydantoin compound described in WO 2015/160488 and the like; an acrylonitrile compound described in WO 2016/105371 and the like; a hemiformal and a hemiacetal described in WO 2017/044248, WO 2017/044250, and the like; a composition containing acetic anhydride and a triacetamide described in U.S. Patent No. 4,909,925 and the like; a carboxylate compound described in U.S. Patent No. 4,802,973 and the like; an acrylate compound described in WO 2016/105370 and the like; and a hydrogen sulfide removing agent product described in British Patent Application Publication No. 2,446,915. The sulfur-containing compound absorbing agent may be used solely or as a combination of two or more kinds thereof.

Among the aforementioned compounds, at least one compound selected from the group consisting of an α,β-unsaturated aldehyde and a dialdehyde is preferred, and at least one compound selected from the group consisting of an α,β-unsaturated aldehyde having 3 to 10 carbon atoms and a dialdehyde having 6 to 20 carbon atoms is preferred, and at least one compound selected from the group consisting of acrolein, senecioaldehyde, 3-methylglutaraldehyde, 1,9-nonanedial, and 2-methyl-1,8-octanedial is further preferred, from the standpoint of absorption efficiency of the sulfur-containing compound, and at least one compound selected from the group consisting of senecioaldehyde, 1,9-nonanedial, and 2-methyl-1,8-octanedial is particularly preferred from the standpoint of the low toxicity, the biodegradability, the handling safety, the heat resistance, the low metal corrosion property, and the like.

At least one compound selected from the group consisting of 1,9-nonanedial and 2-methyl-1,8-octanedial is preferred from the standpoint that the compound has high hydrophobicity and can be readily mixed with the non-aqueous liquid after use.

From the standpoint of absorption efficiency of the sulfur-containing compound, the total amount of the α,β-unsaturated aldehyde and the dialdehyde in the sulfur-containing compound absorbing agent is preferably not less than 5% by mass, more preferably not less than 10% by mass, and further preferably not less than 20% by mass. In addition, the above total amount is preferably 100% by mass or less, for example, 80% by mass or less. Further, in one embodiment, the sulfur-containing compound absorbing agent may consist of the α,β-unsaturated aldehyde and the dialdehyde.

In addition, from the standpoint of absorption efficiency of the sulfur-containing compound, the content of α, β-unsaturated aldehyde in the sulfur-containing compound absorbing agent is preferably not less than 5% by mass, more preferably not less than 10% by mass, and further preferably not less than 20% by mass. Further, the above content is preferably 100% by mass or less, for example, 80% by mass or less. Additionally, in one embodiment, the sulfur-containing compound absorbing agent may consist of only the α,β-unsaturated aldehyde.

From the standpoint of absorption efficiency of the sulfur-containing compound, the total amount of the α,β-unsaturated aldehyde and the dialdehyde in the total amount of active ingredients of the sulfur-containing compound absorbing agent is preferably not less than 40% by mass, more preferably not less than 50% by mass, and further preferably not less than 60% by mass. Further, the above total amount is preferably 100% by mass or less, for example, 80% by mass or less. Additionally, in one embodiment, the sulfur-containing compound absorbing agent may consist of only the α,β-unsaturated aldehyde and the dialdehyde.

In addition, from the standpoint of absorption efficiency of the sulfur-containing compound, the content of α, β-unsaturated aldehyde in the total amount of active ingredients of the sulfur-containing compound absorbing agent is preferably not less than 40% by mass, more preferably not less than 50% by mass, and particularly preferably not less than 60% by mass. Further, the above content is preferably 100% by mass or less, for example, 80% by mass or less. Additionally, in one embodiment, the sulfur-containing compound absorbing agent may consist of only the α,β-unsaturated aldehyde.

In the case where at least one compound selected from the group consisting of 1,9-nonanedial and 2-methyl-1,8-octanedial is used as the sulfur-containing compound absorbing agent, 1,9-nonanedial or 2-methyl-1,8-octanedial may be used solely, and a mixture of 1,9-nonanedial and 2-methyl-1,8-octanedial is preferably used from the standpoint of the industrial availability. The mixing ratio of the mixture of 1,9-nonanedial and 2-methyl-1,8-octanedial is not particularly limited, and in general, the mass ratio 1,9-nonanedial/2-methyl-1,8-octanedial is preferably from 99/1 to 1/99, more preferably from 95/5 to 5/95, further preferably from 93/7 to 45/55, and particularly preferably from 90/10 to 55/45.

The sulfur-containing compound absorbing agent may contain an organic compound in such a range that does not impair the effects. Examples of the organic compound include a polyalkylene glycol, such as polyethylene glycol, polypropylene glycol, and polybutylene glycol, described in WO 2016/121747, and a polyamine (1) represented by the following general formula (1).

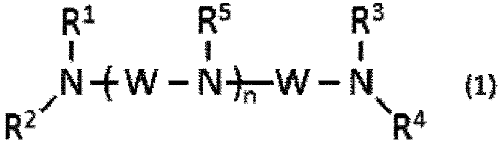

In the general formula (1), W represents a methylene group, a dimethylene group, or a trimethylene group; R¹ to R⁵ each independently represent a hydrogen atom, an alkyl group having from 1 to 6 carbon atoms, an alkenyl group having from 1 to 6 carbon atoms, an aralkyl group having from 7 to 20 carbon atoms, or an aryl group having from 6 to 12 carbon atoms; and n represents an integer of from 0 to 3,000, provided that R¹ and R², and R³ and R⁴ each independently may be bonded to each other to form a ring (i.e., only R¹ and R² may be bonded to form a ring, only R³ and R⁴ may be bonded to form a ring, or R¹ and R², and R³ and R⁴ each may be bonded to each other to form a ring); W may be substituted with one or more groups each represented by R⁶ (wherein R⁶ represents an alkyl group having from 1 to 6 carbon atoms, an alkenyl group having from 1 to 6 carbon atoms, an aralkyl group having from 7 to 20 carbon atoms, or an aryl group having from 6 to 12 carbon atoms); R¹ to R⁶ each may be substituted with a hydroxyl group or an alkoxy group having from 1 to 6 carbon atoms; and in the case where plural groups represented by W, R⁵, or R⁶ exist, the groups may not be necessarily the same as each other.

In the polyamine (1), W preferably represents a dimethylene group or a trimethylene group, and more preferably a dimethylene group, and it is particularly preferred that all the groups represented by W existing in the general formula (1) are dimethylene groups.

R¹ to R⁵ each preferably represent a hydrogen atom or an alkyl group having from 1 to 6 carbon atoms, more preferably a hydrogen atom or an alkyl group having from 1 to 4 carbon atoms, and further preferably a hydrogen atom or a methyl group.

n preferably represents an integer of from 0 to 1,000, more preferably an integer of from 0 to 100, further preferably an integer of from 0 to 10, and particularly preferably an integer of from 0 to 5.

Examples of the polyamine (1), in which n = 0, include:
a compound, in which W is a methylene group, such as N,N,N',N'-tetramethyldiaminomethane;
a compound, in which W is a dimethylene group, such as ethylenediamine, 1,2-diaminopropane, 1,2-diamino-2-methylpropane, N-methylethylenediamine, N-ethylethylenediamine, N-propylethylenediamine, N-butylethylenediamine, N-benzylethylenediamine, N-phenylethylenediamine, N,N-dimethylethylenediamine, N,N-diethylethylenediamine, N,N-dipropylethylenediamine, N,N-dibutylethylenediamie, N,N'-dimethylethylenediamine, N,N'-diethylethylenediamine, N,N'-dipropylethylenediamine, N,N'-diisopropylethylenediamine, N,N'-di-tert-butylethylenediamine, N,N'-dibenzylethylenediamine, N,N'-diphenylethylenediamine, N,N'-bis(2-hydroxyethyl)ethylenediamine, N,N,N'-trimethylethylenediamine, N,N,N'-triethylethylenediamine, N,N,-diethyl-N'-methylethylenediamine, N,N,N',N'-tetramethylethylenediamine, N,N,N',N'-tetraethylethylenediamine, N,N,N',N'-tetraisopropylethylenediamine, and N,N,N',N'-tetramethyl-1,2-diaminopropane; and
a compound, in which W is a trimethylene group, such as 1,3-propanediamine, 2-methyl-1,3-propanediamine, 1,3-diaminobutane, 2,2-dimethyl-1,3-propanediamine, 1,3-diaminopentane, N-methyl-1,3-propanediamine, N-ethyl-1,3-propanediamine, N-propyl-1,3-propanediamine, N-butyl-1,3-propanediamine, N-benzyl-1,3-propanediamine, N-phenyl-1,3-propanediamine, N,N-dimethyl-1,3-propanediamine, N,N-diethyl-1,3-propanediamine, N,N-dipropyl-1,3-propanediamine, N,N'-dimethyl-1,3-propanediamine, N,N'-diethyl-1,3-propanediamine, N,N,N'-trimethyl-1,3-propanediamine, N,N,N',N'-tetramethyl-1,3-propanediamine, N,2-dimethyl-1,3-propanediamine, and N,N,2,2-tetramethyl-1,3-propanediamine.

Examples of the polyamine (1), in which n = 1, include:
a compound, in which W is a dimethylene group, such as diethylenetriamine, 2,2'-diamino-N-methyldiethylamine, N,N',N"-trimethyldiethylenetriamine, N,N,N',N",N"-pentamethyldiethylenetriamine, and N,N,N',N'-tetrabutyldiethylenetriamine; and
a compound, in which W is a trimethylene group, such as 3,3'-diaminodipropylamine and N,N-bis(3-(dimethylamino)propyl)amine.

Examples of the polyamine (1), in which n ≥ 2, include:
a compound, in which n = 2, such as triethylenetetramine and 1,1,4,7,10,10-hexamethyltriethylenetetramine;
a compound, in which n = 3, such as tetraethylenepentamine; and
a compound, in which n = 4, such as pentaethylenehexamine.

Among the aforementioned compounds, from the standpoint of the availability and the effect of removing a sulfur-containing compound, ethylenediamine, N-methylethylenediamine, N,N-dimethylethylenediamine, N,N'-dimethylethylenediamine, N,N,N'-trimethylethylenediamine, N,N,N',N'-tetramethylethylenediamine, diethylenetriamine, 2,2'-diamino-N-methyldiethylamine, N,N,N',N",N"-pentamethyldiethylenetriamine, and triethylenetetramine are preferred, and ethylenediamine, N-methylethylenediamine, N,N-dimethylethylenediamine, N,N'-dimethylethylenediamine, N,N,N'-trimethylethylenediamine, N,N,N',N'-tetramethylethylenediamine, and diethylenetriamine are more preferred.

The polyamine (1) may be used solely or as a combination of two or more kinds thereof.

The polyamine (1) used may be a commercially available product or may be produced by a known method, such as reaction of ammonia and an alkyl halide.

In the case where the sulfur-containing compound absorbing agent contains at least one of polyalkylene glycol and polyamine (1) represented by the above general formula (1), the total amount of the polyalkylene glycol and the polyamine (1) represented by the general formula (1) in the total amount of active ingredients of the sulfur-containing compound absorbing agent is, for example, 1 to 50% by mass, and in one embodiment, 10 to 40% by mass.

In addition, the total amount of the polyalkylene glycol, the polyamine (1) represented by the general formula (1), α,β-unsaturated aldehyde and dialdehyde in the total amount of active ingredients of the sulfur-containing compound absorbing agent is preferably 60 to 100% by mass, more preferably 80 to 100% by mass, further preferably 90 to 100% by mass and particularly preferably 95 to 100% by mass.

In addition, the total amount of the polyalkylene glycol and the polyamine (1) represented by the general formula (1) is preferably 10 to 100 parts by mass, and more preferably 30 to 60 parts by mass, based on 100 parts by mass of the total amount of the α, β-unsaturated aldehyde and the dialdehyde.

In addition, in the case where the sulfur-containing compound absorbing agent contains polyamine (1) represented by the above general formula (1), the content of the polyamine (1) represented by the general formula (1) in the sulfur-containing compound absorbing agent is, for example, 1 to 50% by mass, and in one embodiment, 10 to 40% by mass.

In addition, the content of the polyamine (1) represented by the general formula (1) is preferably 10 to 100 parts by mass, and more preferably 30 to 60 parts by mass, based on 100 parts by mass of the content of the α, β-unsaturated aldehyde.

The sulfur-containing compound absorbing agent may further contain an arbitrary component, such as a surfactant, a corrosion inhibitor, a deoxidizing agent, an iron controlling agent, a flocculating agent, a temperature stabilizer, a pH controlling agent, a dehydration controlling agent, a scale inhibitor, a biocide, a defoaming agent, a lubricant, a nitrogen-containing compound other than the polyamine (1), and a solvent.

The sulfur-containing compound removing part of bringing the gas to be treated containing a sulfur-containing compound into contact with the oil-soluble sulfur-containing compound absorbing agent, so as to remove the sulfur-containing compound is not particularly limited, as far as the part has such a structure that the gas to be treated can be sufficiently brought into contact with the sulfur-containing compound absorbing agent. Examples of the sulfur-containing compound removing part used include a packed column, a spray column, a scrubber, a bubble column, a bubble agitation tank, a static mixer, a forced vibration bubble column, a vertical rising thin film device, and an orifice pipe device. Among these, a packed column and a bubble column are preferred from the standpoint of the easiness in device design and the small required power. In the case where a packed column is used, the sulfur-containing compound absorbing agent is supplied to the upper part of the column with a supplying device, such as a pump, and is brought into contact with the gas to be treated. The sulfur-containing compound absorbing agent having absorbed the sulfur-containing compound (the sulfur-containing compound absorbed agent) reaches the lower part of the column and is discharged from the sulfur-containing compound removing part, and at least a part thereof is supplied to the mixing part described later. A part of the discharged sulfur-containing compound absorbed agent may be supplied again to the sulfur-containing compound removing part with a circulating device, such as a pump, and may be used again for absorbing the sulfur-containing compound along with the fresh sulfur-containing compound absorbing agent.

In the sulfur-containing compound removing part, the gas to be treated may be brought into contact with the sulfur-containing compound absorbing agent in such a manner that the amount of the sulfur-containing compound absorbing agent used is preferably from 0.1 to 5,000 parts by mass, and more preferably from 1 to 1,000 parts by mass, based on 1 part by mass of the sulfur-containing compound in the entire amount of the gas to be supplied for the treatment.

The temperature of the sulfur-containing compound removing part is not particularly limited, and is preferably in a range of from -30 to 150°C, and more preferably from 0 to 130°C. The pressure of the sulfur-containing compound removing part is not particularly limited, and is preferably in a range of from -0.1 to 10 MPa, and more preferably from 0 to 1.0 MPa.

The mixing part of mixing the sulfur-containing compound absorbed agent and a non-aqueous liquid is not particularly limited, as far as the part has such a structure that the sulfur-containing compound absorbed agent and the non-aqueous liquid can be sufficiently mixed. Examples of the mixing part used include a batch mixing device and an in-line mixing device.

The non-aqueous liquid is not particularly limited, as far as the non-aqueous liquid dissolves the treated sulfur-containing compound absorbing agent, and examples thereof include: an aliphatic hydrocarbon, such as methane, ethane, propane, butane, pentane, n-hexane, and cyclohexane; an aromatic hydrocarbon, such as benzene, toluene, xylene, mesitylene, and naphthalene; a monoalcohol or a diol, such as methanol, ethanol, 2-propanol, ethylene glycol, and polyethylene glycol; a petroleum product, such as gasoline, naphtha, light oil, heavy oil, and kerosene; biodiesel oil; crude oil; liquefied natural gas; liquefied petroleum gas; and condensate formed in mining of natural gas. Among these, a compound containing a hydrocarbon, such as an aliphatic hydrocarbon and an aromatic hydrocarbon, is preferred, and a compound containing at least one of an aliphatic a hydrocarbon having 1 to 15 carbon atoms and an aromatic hydrocarbon having 6 to 18 ring forming carbon atoms is more preferred. The non-aqueous liquid that is in a gaseous state under ordinary temperature and ordinary pressure may be mixed after being liquefied by controlling the temperature and the pressure. The non-aqueous liquid may be used solely or as a mixture of two or more kinds thereof.

In the case where the apparatus for removing a sulfur-containing compound according to the present invention is used in a mining site of crude oil or natural gas, a liquid that is generated on the mining of crude oil or natural gas is most preferably used as the non-aqueous liquid since the non-aqueous liquid may not be necessarily separately acquired. Specific examples of the liquid include crude oil, liquefied natural gas, liquefied petroleum gas, and condensate formed in mining of natural gas.

In the case where crude oil is used as the non-aqueous liquid, for example, the sulfur component contained in the sulfur-containing compound absorbed agent is treated along with other sulfur components contained in the crude oil with a sulfur component recovery apparatus provided in a refinery or the like. Consequently, there is no necessity of building a large-scale treatment apparatus in the mining site of crude oil or natural gas, and the waste materials can be minimized.

Accordingly, in the apparatus for removing a sulfur-containing compound according to the present invention, the utilities required for the treatment and the waste materials can be reduced by treating the sulfur-containing compound absorbed agent by mixing with a non-aqueous liquid.

For example, the apparatus for removing a sulfur-containing compound according to the invention, includes:
a sulfur-containing compound removing part of bringing a gas to be treated containing a sulfur-containing compound into contact with an oil-soluble sulfur-containing compound absorbing agent, so as to remove the sulfur-containing compound;
a mixing part of mixing a sulfur-containing compound absorbed agent resulted from having the sulfur-containing compound absorbed to the sulfur-containing compound absorbing agent, and a non-aqueous liquid; and
a supplying device of supplying the gas to be treated being discharged from a mining site of crude oil or natural gas, or a refinery of crude oil or natural gas to the sulfur-containing compound removing part, and wherein the mixing part is included in a sulfur component recovery apparatus provided in the mining site or the refinery.

### Examples

The present invention will be described in more detail with reference to examples and the like below, but the present invention is not limited to the examples.

### Production Example 1

### Production of Mixture of 1,9-Nonanedial (NL) and 2-Methyl-1,8-octanedial (MOL)

A mixture of 1,9-nonanedial (which is hereinafter referred to as NL) and 2-methyl-1,8-octanedial (which is hereinafter referred to as MOL) was produced (the mixture is hereinafter referred to as NL/MOL) according to the method described in Japanese Patent No. 2,857,055. The mass ratio of NL and MOL in the mixture was NL/MOL = 85/15.

### Test Example 1

### Preparation of Hydrogen Sulfide-absorbing Liquid

In a 50-mL three-neck flask, 10 g of NL/MOL and 5 g of N,N,N',N'-tetramethylethylenediamine (produced by Wako Pure Chemical Industries, Ltd.) were charged and well mixed. A mixed gas having a composition containing 1% by volume of hydrogen sulfide and 99% by volume of nitrogen was passed through the mixed liquid at a flow rate of 50 mL/min for 3 hours, so as to provide a reaction liquid having absorbed therein hydrogen sulfide.

### Test Example 2

### Preparation of Hydrogen Sulfide-absorbing Liquid

In a 50-mL three-neck flask, 5 g of NL/MOL was charged, and a mixed gas having a composition containing 1% by volume of hydrogen sulfide and 99% by volume of nitrogen was passed therethrough at a flow rate of 50 mL/min for 15 hours, so as to provide a reaction liquid having absorbed therein hydrogen sulfide.

### Example 1

In a 50-mL sample tube, 30 g of crude oil (produced by Japan Petroleum Exploration Co., Ltd.) was charged, and the reaction liquid prepared in Test Example 1 was added thereto in an amount of 2% by mass based on the total, and then mixed. The observation of the state of the mixture revealed that the reaction liquid was completely dissolved to form a uniform mixed liquid.

### Example 2

In a 100-mL hyper glass cylinder, 30 g of propane (produced by Suzuki Shokan Co., Ltd.) was charged, and the reaction liquid prepared in Test Example 1 was added thereto in an amount of 0.5% by mass based on the total, and then mixed. The observation of the state of the mixture revealed that the reaction liquid was completely dissolved to form a uniform mixed liquid.

### Example 3

In a 50-mL sample tube, 30 g of solvent naphtha (produced by Wako Pure Chemical Industries, Ltd.) was charged, and the reaction liquid prepared in Test Example 1 was added thereto in an amount of 5% by mass based on the total, and then mixed. The observation of the state of the mixture revealed that the reaction liquid was completely dissolved to form a uniform mixed liquid.

### Example 4

In a 50-mL sample tube, 30 g of crude oil (produced by Japan Petroleum Exploration Co., Ltd.) was charged, and the reaction liquid prepared in Test Example 2 was added thereto in an amount of 2% by mass based on the total, and then mixed. The observation of the state of the mixture revealed that the reaction liquid was completely dissolved to form a uniform mixed liquid.

It is understood from the aforementioned results that the sulfur-containing compound absorbed agent and the non-aqueous liquid are sufficiently mixed, and thus the object of the present invention has been achieved by using the apparatus for removing a sulfur-containing compound according to the present invention.

### Industrial Applicability

The apparatus for removing a sulfur-containing compound of the present invention and the method for removing a sulfur-containing compound using the apparatus are useful since the utilities required for the treatment and the waste materials can be reduced in the removal of a sulfur-containing compound contained in a gas.

## Claims

1. An apparatus for removing a sulfur-containing compound, comprising:
a sulfur-containing compound removing part of bringing a gas to be treated containing a sulfur-containing compound into contact with an oil-soluble sulfur-containing compound absorbing agent, so as to remove the sulfur-containing compound; and
a mixing part of mixing a sulfur-containing compound absorbed agent resulted from having the sulfur-containing compound absorbed to the sulfur-containing compound absorbing agent, and a non-aqueous liquid.

2. The apparatus for removing a sulfur-containing compound according to claim 1, wherein the non-aqueous liquid comprises a hydrocarbon.

3. The apparatus for removing a sulfur-containing compound according to claim 1 or 2, wherein the non-aqueous liquid is a liquid that is generated on mining of crude oil or natural gas.

4. The apparatus for removing a sulfur-containing compound according to any one of claims 1 to 3, wherein the gas to be treated is a hydrocarbon that is in a gaseous state at ordinary temperature.

5. The apparatus for removing a sulfur-containing compound according to any one of claims 1 to 4, additionally comprising a supplying device of supplying the gas to be treated being discharged from a mining site of crude oil or natural gas, or a refinery of crude oil or natural gas to the sulfur-containing compound removing part, and wherein the mixing part is included in a sulfur component recovery apparatus provided in the mining site or the refinery.

6. A method for removing a sulfur-containing compound, comprising: removing a sulfur-containing compound from a gas to be treated containing the sulfur-containing compound, with the apparatus for removing a sulfur-containing compound according to any one of claims 1 to 5.
